Europäisches Patentamt

European Patent Office

Office européen des brevets

(19)

(11) Publication number: **0 057 507**
**A1**

(12) # EUROPEAN PATENT APPLICATION

(21) Application number: **82300148.2**

(22) Date of filing: **12.01.82**

(51) Int. Cl.³: **C 07 C 143/08,** C 07 C **139/00,**
B 01 J **31/02**

(30) Priority: **29.01.81 GB 8102662**
**27.04.81 GB 8112926**

(43) Date of publication of application: **11.08.82**
**Bulletin 82/32**

(84) Designated Contracting States: **BE CH DE FR GB IT LI**
**NL SE**

(71) Applicant: **IMPERIAL CHEMICAL INDUSTRIES PLC,**
**Imperial Chemical House Millbank, London SW1P 3JF**
**(GB)**

(72) Inventor: **Brown, Geoffrey Teasdale, 215 Birchfield Road,**
**Widnes Cheshire (GB)**
Inventor: **Bowden, Roy Dennis, 5 Red Lane, Frodsham**
**Cheshire (GB)**

(74) Representative: **Roberts, John Stanley et al, IMPERIAL**
**CHEMICAL INDUSTRIES PLC Legal Department:**
**Patents Thames House North Millbank, London**
**SW1P 4QG (GB)**

(54) **Difluoromethanesulphonic acid.**

(57) Difluoromethanesulphonic acid (useful as an acid cata-
lyst) in liquid form, in the form of an aqueous solution thereof or
in the form of the hydrate.

Difluoromethanesulphonic acid may be prepared from
chlorodifluoromethane via an alkali-metal difluoromethanesul-
phonate.

EP 0 057 507 A1

DIFLUOROMETHANESULPHONIC ACID

1.                    MD 31669

This invention relates to difluoromethanesulphonic acid and the preparation and use thereof.

Trifluoromethanesulphonic acid, $CF_3SO_3H$, and pentafluoroethanesulphonic acid, $CF_3CF_2SO_3H$ are the lowest members of a series of perfluoroalkanesulphonic acids described in the specification of United Kingdom Patent 758,467. The route described for the preparation of the perfluoroalkanesulphonic acids involves as the first stage the preparation of the corresponding sulphonic acid fluoride by electrolysing a mixture of anhydrous liquid hydrogen fluoride and a hydrocarbon sulphonic acid.

The reactions and uses of trifluoromethanesulphonic acid (sometimes known by the trivial name "triflic acid") are reviewed by Howells et al in Chemical Reviews, 1977, Vol 77, page 69 onwards. Trifluoromethanesulphonic acid is therein described as having been used in a variety of Friedel-Crafts reactions, including alkylation of benzene and other aromatic hydrocarbons and isomerisation and cracking of alkanes or alkyl-substituted aromatic hydrocarbons, and also in cationic polymerisation and other reactions in which a strong acid catalyst is required.

The use of trifluoromethanesulphonic acid in promoting the hydrogen fluoride catalysed alkylation of isoparaffins having 4 or 5 carbon atoms with mono-olefins having from 3 to 6 carbon atoms is described in US Patent 4 118 433.'

In US Patent 3 370 721 there is described a liquid-phase alkylation process for the production of alkylate useful as motor fuel wherein low molecular weight isoparaffins are alkylated with olefin hydrocarbons in the presence of a catalyst comprising a mixture of a major proportion of sulphuric acid with a minor proportion of trifluoromethane sulphonic acid.

In Canadian Patent 1 093581 there is described a process for the production of chlorofluorinated sulphonic acids by the catalytic oxychlorofluorination of certain sulphur-containing starting materials. Difluoromethanesulphonic acid, $CF_2HSO_3H$, is listed as one of eight halomethanesulphonic acids detected by gas chromatographic analysis of the effluent from the gas-phase reaction between di-(monochloromethyl) sulphide, HF and oxygen;' $CF_2HSO_3H$ is also listed as one of the five halomethanesulphonic acids detected in the effluent from the corresponding reaction of di(monochloromonofluoromethyl) sulphoxide. In neither case is the proportion of $CF_2HSO_3H$ stated;' there is no description of the isolation, properties or uses of this compound.

We have now found that difluoromethanesulphonic acid, $CF_2HSO_3H$, is a sufficiently strong acid to be effective as a catalyst or a promoter in contexts in which trifluoromethanesulphonic acid is known to be useful. Difluoromethanesulphonic acid has a higher boiling-point than trifluoromethanesulphonic acid, and consequently can usually be separated more readily from the products of organic reactions in which it is used as acid catalyst. The greater degree of hydrogen-bonding in difluoromethanesulphonic acid also favours the separation of this compound from the organic phase of a reaction product mixture. Furthermore, difluoromethanesulphonic acid may conveniently be prepared in substantially pure liquid form from chlorodifluoromethane, a readily available starting material, by a method not involving electrolytic fluorination (as is required for trifluoromethanesulphonic acid);' the preparation of difluoromethanesulphonic acid is therefore more economically attractive.

According to the present invention there is provided difluoromethanesulphonic acid $CF_2HSO_3H$ in liquid form, in the form of an aqueous solution thereof or in the form of its hydrate.

Another aspect of the invention consists in the use of difluoromethanesulphonic acid as an acid catalyst or as a promoter of an acid catalyst.

Difluoromethanesulphonic acid, the compound of the present invention, may be prepared from an alkali-metal difluoromethanesulphonate, by heating with a mineral acid, for example sulphuric acid, and distilling off the free difluoromethanesulphonic acid from the reaction mixture under reduced pressure;' preferably 98%-100% sulphuric acid is used in this process.

The preparation of sodium difluoromethanesulphonate by the reaction between chlorodifluoromethane and aqueous sodium sulphite heptahydrate is described by Farrar (J. Chem. Soc 1960, page 3062) as a stage in the preparation of difluoro-methanesulphonanilide and other n-aryl-difluoro-methanesulphonamides.

There are, however, difficulties in the preparation of sodium difluoromethanesulphonate. The erratic and often poor yields in the reaction between $CHF_2Cl$ and sodium sulphite are described by Moore in J.Org.Chem.$\underline{44}$, 1708 (1979).

We have found that difluoromethanesulphonic acid may advantageously be prepared via the potassium salt. The relatively higher solubility of potassium sulphite in water (compared with that of sodium sulphite) allows a relatively higher concentration of sulphite ions to be present in the reaction with chlorodifluoromethane a relatively higher efficiency of conversion of the chlorodifluoromethane to the difluoromethanesulphonate and consequently to the acid may therefore be achieved.

Thus according to another aspect of the present invention there is provided a method of preparation of difluoromethanesulphonic acid which comprises the steps of (a) preparing potassium difluoromethanesulphonate by the reaction of chlorodifluoromethane with aqueous potassium sulphite and (b) heating the potassium difluoromethanesulphonate with a strong mineral acid and distilling off the free difluoromethanesulphonic acid from the reaction mixture under reduced pressure.

The most convenient mineral acid is sulphuric acid but other acids which may be used include orthophosphoric acid and hydrogen fluoride.

Prior to step (b) the potassium difluoromethanesulphonate prepared by step (a) is preferably purified by extraction into an organic solvent, followed by evaporation of the solvent. The preferred solvent for this purpose is acetone (especially aqueous acetone containing about 5% by volume of water);'other solvents which may be used include industrial methylated spirits.

Difluoromethanesulphonic acid may be used, in general, as acid catalyst in reactions of the type in which trifluoromethanesulphonic acid is known to be useful, for example the reactions already referred to herein. The reaction conditions and the proportion of the acid employed (optionally in conjunction with other catalysts) are in general those known in the art in respect of trifluoromethanesulphonic acid.

The invention is illustrated by the following Examples. Parts and percentages are by weight unless otherwise stated.

Difluoromethanesulphonic acid also has potential application as an electrolyte, for example in fuel cells;' the compound is non-wetting towards poly(tetrafluoroethylene) which makes it especially useful in such applications.

EXAMPLE 1

Sodium difluoromethanesulphonate was prepared by the reaction between chlorodifluoromethane and sodium sulphite heptahydrate as described by Farrar (op cit). The crude sodium salt product contained about 25% of sodium difluoromethanesulphonate.

The crude sodium salt (50g) was mixed with industrial methylated spirits (600 ml) and the mixture was stirred under reflux for 4 hours. At the end of this period the mixture was cooled and the insoluble material was removed by filtration and the filtrate was evaporated to dryness to yield a solid concentrate (17.8g) containing 70% sodium difluoromethanesulphonate. The dry concentrate was added slowly to 98% sulphuric acid (22 ml) at 40°C-50°C with stirring. Distillation of the mixture gave 9.8g difluoromethanesulphonic acid, boiling-point 71°C-79°C at 0.3-0.4 m bar. Redistillation gave difluoromethanesulphonic acid as a colourless hygroscopic liquid, boiling-point 75°C at 1 m bar, density 1.72 g/ml at 25°C.

Difluoromethanesulphonic acid forms a monohydrate, melting point 55°C, which is a white, crystalline deliquescent solid;' the monohydrate can be distilled unchanged from sulphuric acid.

The acid strength ($pK_a$) of difluoromethanesulphonic acid was measured and found to be 5.7. (By way of comparison $pK_a$ for trifluoromethanesulphonic acid is 4.7 and $pK_a$ for sulphuric acid is 7.0).

Difluoromethanesulphonic acid, $CF_2HSO_3H$ was characterised by nuclear magnetic resonance and by infra-red spectrum as follows.

Nuclear magnetic resonance

Recorded using a Jeol FX100 Fourier transform spectrometer.

$^1H$ and $^{19}F$ spectra were recorded at 100 MHz and 93.7 MHz respectively. Proton chemical shifts are quoted in ppm to low field of tetramethylsilane internal standard. Fluorine chemical shifts are quoted in ppm to high field of trichloro-fluoromethane internal standard.

$$CF_2HSO_3H$$
$$1\ 2\quad 3$$

Chemical shift

$$^{19}F\qquad\qquad\qquad ^{1}H$$

1. 121.2 (d)     2. 6.68 (t)     3. 10.56 (t)

Coupling constant

$J (1,2)$ 52.5 $H_z$

Infra-red (KBr disc) The main peaks were as follows:

1045 $cm^{-1}$, 1060 $cm^{-1}$ (s, doublet);' 1240 $cm^{-1}$ (s, broad) with shoulder at 1322 $cm^{-1}$ (s);'

3000 $cm^{-1}$ (w)

EXAMPLE 2

This Example shows the use of difluoromethanesulphonic acid as a catalyst for the alkylation of toluene by propene.

A mixture of propene (5.6 volumes) and nitrogen (1 volume) was circulated through a mixture of 22g dry toluene and 0.74g difluoromethanesulphonic acid (0.024 mole/mole toluene) in a closed system at a rate of about 100l/hour, the temperature of the liquid being held at $105\pm2°C$. As the propene reacted the pressure in the system was restored by introducing more propene from a cylinder. After 30 minutes the gas flow was stopped and the liquid washed with water and analysed by gas-liquid chromatography. The propene consumed in this time was 1.01 mole per mole of toluene and the alkylates produced comprised 51.2% monoisopropyltoluenes, 47.2% diisopropyl toluenes and 1.6% triisopropyl toluenes.

EXAMPLE 3

This Example shows the use of difluoromethanesulphonic acid as a catalyst for the alkylation of p-xylene by ethylene.

A mixture of ethylene (5.6 volumes) and nitrogen (1 volume) was circulated through a mixture of 21.5g p-xylene and 1.0g difluoromethanesulphonic acid (0.037 mole difluoromethanesulphonic acid per mole of p-xylene) at 130°C. The pressure was restored with ethylene as the ethylene reacted. The reaction was allowed to proceed for 6 hours, after which the gas flow was stopped and the liquid washed with water and analysed by gas chromatography. The ethylene consumed amounted to 0.2 mole per mole of xylene and the alkylate comprised 75.4% monoethyl-p-xylene, 17.5% diethyl-p-xylenes and 4.4% triethyl-p-xylenes.

EXAMPLE 4

Potassium difluoromethanesulphonate was prepared by the reaction between chlorodifluoromethane (86.5g, 1 mole) in 500 ml water at 120°C for 5 hours in a sealed vessel. The crude potassium salt product contained about 25% of potassium difluoromethanesulphonate. 57% of the chlorodifluoromethane was consumed and of the chlorodifluoromethane consumed 70% was converted into potassium difluoromethanesulphonate.

The crude potassium salt (50g) was mixed with industrial methylated spirits (500 ml) and the mixture was stirred under reflux for 4 hours. At the end of this period the insoluble material was removed by filtration and the filtrate was evaporated to dryness to yield a solid concentrate (21g) containing 60% potassium difluoromethanesulphonate. The dry concentrate was added slowly to 98% sulphuric acid (23 ml) at 40°C-50°C with stirring. Distillation of the mixture gave 8.9g difluoromethanesulphonic acid, boiling point 71°C-79°C at 0.3-0.4 mbar. Redistillation gave difluoromethanesulphonic acid as a colourless hygroscopic liquid, boiling point 79°C-80°C at 1 mbar density, 1.72 q/ml at 25°C.

EXAMPLE 5

This Example shows the use of difluoromethanesulphonic acid in the alkylation of isobutane.

A stainless steel pressure vessel was cooled to -20°C and charged with isobutane (25 g), cis but-2-ene (2 g), hydrogen fluoride (24 g) and difluoromethanesulphonic acid (3 g).

The contents of the vessel were allowed to warm to 0°C under autogenous pressure and vigorously shaken under these conditions for a period of 5 minutes.

At the end of this period the excess hydrocarbon was slowly vented from the vessel and the liquid products were decanted into a polythene separating funnel. The organic alkylate (the upper layer) was analysed by gas-liquid chromatography and the octane rating of the alkylate was computed by the method described by Hutson and Logan, Hydrocarbon Processing (1975) page 107. The octane rating was found to be 97.4.

By way of comparison, the same procedure was repeated except that the difluoromethanesulphonic acid was omitted and that the reaction period was 10 minutes. The octane rating of the product was found to be 93.4.

CLAIMS

1.  Difluoromethanesulphonic acid in liquid
    form or in the form of an aqueous solution
    thereof.

2.  Substantially pure liquid difluoromethanesulphonic acid.

3.  The hydrate of difluoromethanesulphonic
    acid.

4.  A process for the preparation of difluoromethanesulphonic acid characterised in that
    an alkali-metal difluoromethanesulphonate is
    heated with a strong mineral acid.

5.  A process according to Claim 4 characterised
    in that the mineral acid is sulphuric
    acid.

6.  A process according to Claim 4 or Claim 5
    characterised in that the alkali-metal
    salt is potassium difluoromethanesulphonate.

7.  A process according to any of Claims 4 to
    6 characterised in that the alkali-metal
    difluoromethanesulphonate is prepared by the
    reaction of chlorodifluoromethane with
    aqueous alkali-metal sulphite.

8.  An alkylation process wherein an aromatic
    hydrocarbon or an isoparaffin is reacted
    with an olefinic hydrocarbon in the presence
    of a fluoroalkanesulphonic acid as catalyst
    or promoter characterised in that the
    fluoroalkanesulphonic acid is difluoromethane-
    sulphonic acid.

**European Patent Office**

**EUROPEAN SEARCH REPORT**

0057507

Application number

EP 82 30 0148

## DOCUMENTS CONSIDERED TO BE RELEVANT

| Category | Citation of document with indication, where appropriate, of relevant passages | Relevant to claim |
|---|---|---|
| X | US - A - 4 080 391 (R. TSUMURA et al.)  <br><br> * column 7; example 4 * | 1-3 |
| D/X | CA - A - 1 093 581 (ALLIED CHEMICAL CORP.)  <br><br> * page 27, table III * | 1-3 |
| A | US - A - 2 732 398 (T.J. BRICE)  <br><br> * column 8, lines 28-30 * | 4-6 |

**CLASSIFICATION OF THE APPLICATION (Int. Cl. ³)**

C 07 C 143/08
           139/00
B 01 J  31/02

**TECHNICAL FIELDS SEARCHED (Int.Cl.³)**

C 07 C 143/00

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons
&: member of the same patent family, corresponding document

The present search report has been drawn up for all claims

| Place of search | Date of completion of the search | Examiner |
|---|---|---|
| The Hague | 23-02-1982 | GRAMAGLIA |

EPO Form 1503.1 06.78